# EUROPEAN PATENT SPECIFICATION

(11) **EP 2 098 240 B1**
(45) Date of publication and mention of the grant of the patent: **11.09.2013**
(21) Application number: 07830257.7
(22) Date of filing: 22.10.2007
(51) Int. Cl.: A61K 35/66, C12N 1/14, A61P 17/02, A61P 43/00, C12Q 1/04

(54) **AGENT FOR PROMOTING HEALING OF LIVING BODY**
MITTEL ZUR FÖRDERUNG DER HEILUNG EINES LEBENDEN KÖRPERS
AGENT FAVORISANT LA GUÉRISON D'UN ORGANISME VIVANT

(30) Priority: 02.11.2006 JP 2006298478
(43) Date of publication of application: 09.09.2009
(60) Divisional application: 13168694.1
(73) Proprietor: Aureo Co., Ltd., Tokyo 108-0071 (JP)
(72) Inventor: MORIYA, Naoyuki, Tokyo 1080071 (JP); MORIYA, Yukiko, Tokyo 1080071 (JP); KUBOTA, Koji, Tokyo 1080071 (JP)
(74) Representative: Beacham, Annabel Rose
(86) International application number: PCT/JP2007/070523
(87) International publication number: WO 2008/053728

(56) References cited:
- EP-A1- 1 602 377
- JP-A- 11 313 667
- JP-A- 2002 204 687
- JP-A- 2002 335 926
- JP-A- 2003 113 097
- JP-A- 2004 269 407
- JP-A- 2006 075 076
- JP-A- 2006 345 827
- MUHAMMAD WASIF SAIF ET AL: "5-Fluorouracil dose escalation enabled with PN401 (triacetyluridine): toxicity reduction and increased antitumor activity in mice" CANCER CHEMOTHERAPY AND PHARMACOLOGY, SPRINGER, BERLIN, DE LNKD- DOI:10.1007/S00280-005-0129-X, vol. 58, no. 1, 1 July 2006 (2006-07-01), pages 136-142, XP019334368 ISSN: 1432-0843

## Description

### BACKGROUND OF THE INVENTION

### 1. Field of the Invention

The present invention relates to a living body healing accelerator containing as an active constituent a mixture of a culture solution obtained after cultivation of a microorganism belonging to the *Aureobasidium sp*. and mycelia of the microorganism cultivated in the culture solution.

### 2. Description of the Related Art

A culture solution obtained after cultivation of a microorganism belonging to the *Aureobasidium sp*. is now used as a food or beverage product or as a food additive. The culture solution contains β-glucan, and it is known that the β-glucan is 1,3,1,6-β-D-glucan having a glucose residue with β-1,3 linkage as a principal chain and a glucose residue with β-1,6 linkage coupled thereto as a side chain (or a branched chain). (Acta Chemica Scandinavia 17, 1351-1356 (1963) and Agric. Biol. Chem. 47(6), 1167-1172 (1983))

JP 2002-204687 A discloses the fact that a culture solution obtained after cultivation of a microorganism belonging to the *Aureobasidium sp.* shows high anti-tumor activity and high immunostimulation activity when orally administered and can be used as a preventive drug or a curative drug to various types of diseases.

However, it has not been reported yet that the mixture of the culture solution obtained after cultivation of a microorganism belonging to the *Aureobasidium sp.* and mycelia of the microorganism cultivated in the culture solution exhibits an effect on cells and tissues for enhancing their regeneration ability.

JP 2002-204687 A also discloses the fact that the culture solution obtained after cultivation of a microorganism belonging to the *Aureobasidium sp*. can be used as a preventive or curative drug for various types of diseases, and lists diabetes as an example of the diseases. However, JP 2002-204687 A does not disclose any testal data suggesting that the culture solution is active in prevention or therapeutic effect of diabetes.

JP 2002-204687 A also describes that the culture solution obtained after cultivation of a microorganism belonging to the *Aureobasidium sp*. is effective in suppression of proliferation of leukemia cells in an test system using cultured cells, but does not describe any testal data indicating the pharmaceutical effect of the culture solution based on a result of tests with animals.

EP1602377 discloses a composition comprising β-glucan in which the physiologically active effects of β-1,3-1,6-glucan contained in a cultured composition of a bacterium belonging to the genus *Aureobasidium sp.* are further enhanced.

MUHAMMAD WASIF SAIF ET AL: CANCER CHEMOTHERAPY AND PHARMACOLOGY, SPRINGER, BERLIN, DE LNKD- DOI:10.1007/S00280-005-0129-X, vol. 58, no. 1, 1 July 2006 (2006-07-01), pages 136-142, ISSN: 1432-0843 discloses 5-Fluorouracil dose escalation enabled with PN401 (triacetyluridine): toxicity reduction and increased antitumor activity in mice.

Any drug has effects and side effects. When a drug has high effects and low side effects, it is generally considered that the drug has "high safety". However, there are not few drugs, such as anticancer drugs or steroid drugs, which one has to administer even though the drug has relatively strong side effects. This fact reflects a limit of drugs currently available that the toxicity and the effectiveness cannot safely be separated from each other. In such situation, reduction of toxicity of each drug is vitally significant issue.

For instance, in a case of anticancer drugs, even when cells or tissues are damaged due to expression of toxicities such as the activity for suppression of bone morrow, hepatoxicity, and nephrotoxicity and the drug can not be administered more, sometimes a desirable effect may be obtained by continuing administration of the drug a little more. In other words, if toxicity of the anticancer drug can be reduced in such situation, it would be possible to continue administration of the drug in the state where the effectiveness is exhibited to the maximum extent. This is a compelling need in the clinical field where one's life or death must be determined.

Dissection of skin or mucosa performed in association with a surgical operation and injuries caused by burning or laceration can also be regarded as damage to cells or tissues. When dissection is performed, various preventive treatments are performed, such as prevention of infectious diseases caused by bacteria by oral administration of an antibiotic or direct application of the antibiotic drug to an external wound, reduction of inflammation by administration of an antiinflammatory drug, or reduction of pain by administration of an analgetic.

The medical treatment for a wound of skin or mucosa caused by a surgical operation aims only reduction of secondary disturbances such as infection of bacteria, inflammation, and pain. Currently healing of the wound is dependent on skin regeneration ability of the living body's. Because of such circumstances, a long time is required until recovery, and there are still concerns about the possibility of side effects caused by administration of drugs.

To solve the problems described above, active efforts have been made for development of a drug capable of accelerating the ability of healing that a living body originally has, but there is still not any drug that can sufficiently satisfy all of the requirements in the effect, safety, production cost, and the like.

As described above, there is the strong need for providing a living body healing accelerator capable of accelerating healing of damages to tissues caused by toxicity of an administered drug or a wound such as a burn in the clinical field.

### SUMMARY OF THE INVENTION

An object of the present invention is to provide, for solving the problems so ever, a living body healing accelerator which is capable of accelerating regeneration ability of a living body, leading to healing of damages of cells or tissues caused by toxicity of a drug, having high safety, and also not causing any side effect even administered for a long period of time.

To solve the problems described above, the inventors of the present invention devoted themselves to find out natural materials originated from organisms which has high safety to a human body and are present in abundance in nature as natural resources. During the research process, the inventors found that (1) a mixture of a culture solution obtained after cultivation of a microorganism belonging to the *Aureobasidium sp.* and mycelia of the microorganism cultivated in the culture solution shows an accelerating activity in the regeneration ability relating to the cure of damages of cells or tissues caused by toxicity of a drug or a wound in a living body, that (2) the mixture shows the activity of suppressing rise of a blood glucose level caused by diabetes, and that (3) the mixture gives the life prolongation effect to a mouse with leukemia-originated cancer cells implanted therein. Based on the facts described above, the inventors found that the mixture is useful as a living body healing accelerator, and completed the present invention.

According to the present invention, provided is a living body healing accelerator including as an active constituent a mixture of a culture solution obtained after cultivation of a microorganism belonging to the *Aureobasidium sp.* and mycelia of the microorganism cultivated in the culture solution, for use *in vivo* in healing damage to tissue caused by an anticancer drug, wherein the anticancer drug is 5-fluorouracil (5-FU).

According to another embodiment of the present invention, provided is a use of a mixture of a culture solution obtained after cultivation of a microorganism belonging to the *Aureobasidium sp.* and mycelia of the microorganism cultivated in the culture solution in manufacturing a medicament for use *in vivo* in healing damage to tissue caused by an anticancer drug, wherein the anticancer drug is 5-fluorouracil (5-FU).

Further, in the present invention, the microorganism belonging to the *Aureobasidium sp.* is preferably the *Aureobasidium pullulans* strain M-1, deposited number of FERM BP-08615 at International Patent Organism Depositary, National Institute of Advanced Industrial Science and Technology (Independent Administrative Corporation) or the *Aureobasidium pullulans* strain M-2, deposited number of FERM BP-10014 at International Patent Organism Depositary, National Institute of Advanced Industrial Science and Technology (Independent Administrative Corporation).

With the present invention, it is possible to provide a living body healing accelerator containing as an active constituent a mixture of a culture solution obtained after cultivation of a microorganism belonging to the *Aureobasidium sp.* and mycelia of the microorganism cultivated in the culture solution, and damage to tissues caused by the anticancer drug 5-FU can be reduced when the living body healing accelerator is administered concurrently.

As described above, with the living body healing accelerator according to the present invention, it is possible to accelerate activity in the regeneration ability relating to healing of damages of skin and tissues in a living body.

### BRIEF DESCRIPTION OF THE DRAWINGS

Fig. 1 is a view showing change in a volume of a tumor;
Fig. 2 is a view showing change in a body weight;
Fig. 3 is a view showing change in the number of leukocytes;
Fig. 4 is a view illustrating the effect of a Aureobasidium culture solution on reduction of a weight of an organ.

### DETAILED DESCRIPTION OF THE INVENTION

The present invention is described in detail below.

The term of "microorganism belonging to the *Aureobasidium sp*." as used herein is applicable to any strain separated from the environment (such as food products, soil, or indoor environment).

The most preferable example of the microorganism belonging to the *Aureobasidium sp.* is strains belonging to *Aureobasidium pullulans.* More specifically, the preferable microorganisms include strains stocked as single strains respectively such as IFO 4464, IFO 4466, IFO 6353, IFO 7757, ATCC 9348, ATCC 3092, ATCC 42023, and ATCC 433023. Especially, the *Aureobasidium pullulans* strain M-1 (Deposited number of FERM BP-08615 at International Patent Organism Depositary, National Institute of Advanced Industrial Science and Technology (Independent Administrative Corporation)) is the most preferable one because of the high yield of 1,3,1,6-β-D-glucan.

It is also possible to use a mutated strain, which is prepared by subjecting a stock strain separated as a single strain to the ordinary technology for mutation. The ordinary technology for mutation as used herein include, but not limited to UV radiation, or chemical processing with such chemicals as nitrosoguanidine, ethidium bromide, ethyl methanesulfonate, or sodium nitrite, and especially the *Aureobasidium pullulans* strain M-2 (Deposited number of FERM BP-10014 at International Patent Organism Depositary, National Institute of Advanced Industrial Science and Technology (Independent Administrative Corporation)) is the most preferable one because of the high yield of 1,3,1,6-β-D-glucan, and also because accumulation of color in a culture solution is lower as compared to the parent strain M-1.

"A mixture of a culture solution obtained after cultivation of a microorganism belonging to the *Aureobasidium sp.* and mycelia of the microorganism cultivated in the culture solution" according to the present invention is obtained by culturing microorganisms belonging to the *Aureobasidium sp..* The microorganism belonging to the *Aureobasidium sp.* can be cultured according to any of known methods (Refer to, for instance, Japanese Patent Laid-open Publication No. Sho 57-149301). That is, the culture may require inoculating bacteria in a culture medium (with the pH of 5.2 to 6.0) containing a 0.5 to 1.0 mass% of carbon source (such as sucrose), 0.1 mass% of a nitrogen source, and extremely small amounts of other substances (such as vitamins, or inorganic materials), and cultivating the bacteria for 2 to 3 days at a temperature in the range from 20 to 30 °C in the aerated state, preferably in the aerated state with agitation. When β-1,3-1,6-gulcan produces, viscosity of the culture solution becomes higher, and a gel-like material having high viscosity is generated. The culture solution obtained as described above generally contains 0.6 to 1.8 mass% of a solid phase, and 5 to 80 mass% of the β-1,3-1,6-gulcan is contained in the solid phase. In the present invention, it is preferable to use a culture containing 1 mass% or more of β-1,3-1,6-gulcan of the solid phase, and is more preferable to use a culture containing 5 mass% or more of β-1,3-1,6-gulcan in the solid phase. When a concentration of β-1,3-1,6-gulcan in the culture is too low, the sufficient physiological effect of the glucan can not be expected.

Quantification of β-1,3-1,6-gulcan can be performed according to the method described in Japanese Examined Patent Publication No. Hei 3-48201. That is, after culturing is finished, the culture solution is sterilized and is subjected to centrifugation to remove the fungus bodies. Then a mixture solution of chloroform and butanol is added in the obtained solution by 10 volume % and the resultant mixture solution is shaked (sevage method) and then to centrifugation to remove chloroform and insoluble matters. The operating sequence is repeated twice, and then the solution is subjected to ethanol precipitation to recover precipitated materials. The recovered materials are dissolved in distilled water, and pullulan is decomposed by processing with an enzyme. Then the decomposed materials are subjected to dialysis in distilled water, and the dialysis solution is subjected to ethanol precipitation to recover the precipitated material (β-1,3-1,6-gulcan) and determine the yield.

In the present invention, the culture solution obtained as described above may be used after sterilized by heating or pressurizing, or may be used after condensed or dried according to the necessity. Culture of bacteria belonging to *Aureobasidium sp.* are used as food additives like a thickening stabilizer, and the safety is high.

When the mixture of the culture solution obtained after cultivation of the microorganism belonging to the *Aureobasidium sp.* and mycelia of the microorganism cultivated in the culture solution is condensed, the condensation is preferably performed at low temperature in the depressurized state. The condensation may be performed until the mixture is dried and solidified. In this case, it is more preferably to perform freeze drying or spray drying in the depressurized state. It is to be noted that the mixture may be filtered before condensation and then condensed.

The "mixture of the culture solution obtained after cultivation of the microorganism belonging to the *Aureobasidium sp.* and mycelia of the microorganism cultivated in the culture solution" according to the present invention may be further purified. Chromatography or elution using an ion exchange resin may be employed singly or in combination for purifying the mixture according to the present invention.

When chromatography is employed, any of normal phase chromatography, reversed phase chromatography, high-performance liquid chromatography, centrifugal liquid chromatography, column chromatography, thin-layer chromatography and the like may be used singly or in combination. Conditions for purification, such as a carrier and a solvent for elution may be selected according to the necessity in correspondence to the selected chromatography. For instance, in a case of normal phase chromatography, a chloroform methanol-based solvent may be used, and in reversed phase chromatography, a water-methanol-based solvent may be used.

As the elution method using an ion exchange resin, for instance, the method may be employed in which the eluted liquid is diluted and dissolved in water or lower alcohol, the solution is contacted to the ion exchange resin to be adsorbed therein and then eluted with water or lower alcohol. The lower alcohol used in this method is as described above, and methanol is especially preferable. There is no specific restriction over the ion exchange resin to be used in this method on the condition that the ion exchange resin can be used for purification in this technical field, and for instance, any of a porous and cross-linked polystyrene-based resin having a large mesh-like structure, urbanlite, cellulose, and the like may be used.

The "living body healing accelerator" according to the present invention is used for accelerating regeneration of cells and tissues damaged by the drug 5-FU. The uses include, for instance, acceleration of regeneration of cells or tissues damaged by the anticancer drug, such as suppression of bone morrow, hepatoxicity, and nephrotoxicity.

The "living body healing accelerator" according to the present invention is preferably orally administered.

The "living body healing accelerator" according to the present invention may be sold as it is as a product in the market, but generally various types of constituents for improving the taste or for being formed into a required form are added and blended therein, and further, a flavor is added to prepare a final product.

The constituents added and blended in the "living body healing accelerator" according to the present invention include various types of sugars, emulsifying agents, sweeteners, acidifiers, fruit juices or the like. More specifically, sugars such as glucose, sucrose, fructose, honey; sugar alcohols such as sorbitol, xylitol, erythritol, lactitol, and palatinit; and emulsifying agents such as sucrose fatty acid ester, glycerin sugar fatty acid ester, and lecithin may be used as the constituents. In addition, various types of vitamins such as vitamin A, vitamin B, vitamin C, and vitamin E, or extracts of herb, constituents of cereals, constituents of vegetables, and constituents of milk may be added and blended therein to obtain living body healing accelerator having an excellent taste.

The flavors which may be added to the "living body healing accelerator" according to the present invention include those based on yogurt, berries, oranges, Chinese quince, Japanese basil, citrus, apple, mint, grape, pear, custard cream, peach, melon, banana, tropical, herb, black tea, coffee, and the like, and the flavors may be used singly or in combination. There is no specific restriction over an amount of the added flavor, and the amount of added flavor is preferably in the range from 0.05 to 0.5 mass%, and more preferably in the range from about 0.1 to about 0.3 mass% for providing a good taste.

The "living body healing accelerator" described above can be processed into market products in any states including a solid state, a liquid state, and the like.

The "living body healing accelerator" according to the present invention can be used as various forms of preparations such as granules, tablets, or capsules prepared by any known technology in the field of drug and food production, together with medicinal acceptable salts, diluents, preserving agents, coloring agents, and taste-adjusting agents.

Further, the "living body healing accelerator" according to the present invention can be used for health food. The health food means foods especially aimed for maintaining and promoting welfare and health more positively as compared to ordinary foods. More specifically, the foods include, liquid, semisolid or solid products, such as cookie, rice cracker, jelly, sweet jelly of beans, yogurt, steamed bread, other types of cakes, cold beverage, energy drink, soup, and the like. In addition, the "living body healing accelerator" according to the present invention may be decocted to be a tea form. Further, the living body healing accelerator according to the present invention can be processed into health foods by being added, by means of mixing, applying, or spraying, during the final stage of the production process or into the final products.

A dosage of the "living body healing accelerator" according to the present invention varies according to age, symptom, and other factors of a recipient. For instance, when orally administered, one dosage for an adult is 3 to about 100 g when used in the gel-like state, and 200 mg to about 3 g when used in the powder state. When used as an extract, the dosage is in the range from 50 mg to about 2 g according to a level of purification or a moisture content, and in any case, the accelerator is preferably administered 3 times a day and about 30 minutes before each meal. When used as a health food, an amount of the material to be used does not affect a taste or an appearance of the food, and for instance, when used in the powder state, the dosage is preferably in the range from 1 to about 100 g against 1 kg of the food.

### Examples

The present invention is described below in further detail with reference to examples thereof, but it is to be noted that the present invention is not restricted by the examples in any sense.

In the tests described below, a mixture of a culture solution prepared by culturing the *Aureobasidium pullulans* strain M-2 according to the known method and mycelia therein (with the solid phase contents of 1.5 to 2 mass%; hereinafter referred to as Aureobasidium culture solution) was used.

### (Example 1) (Test for reduction of side effects of anticancer drugs)

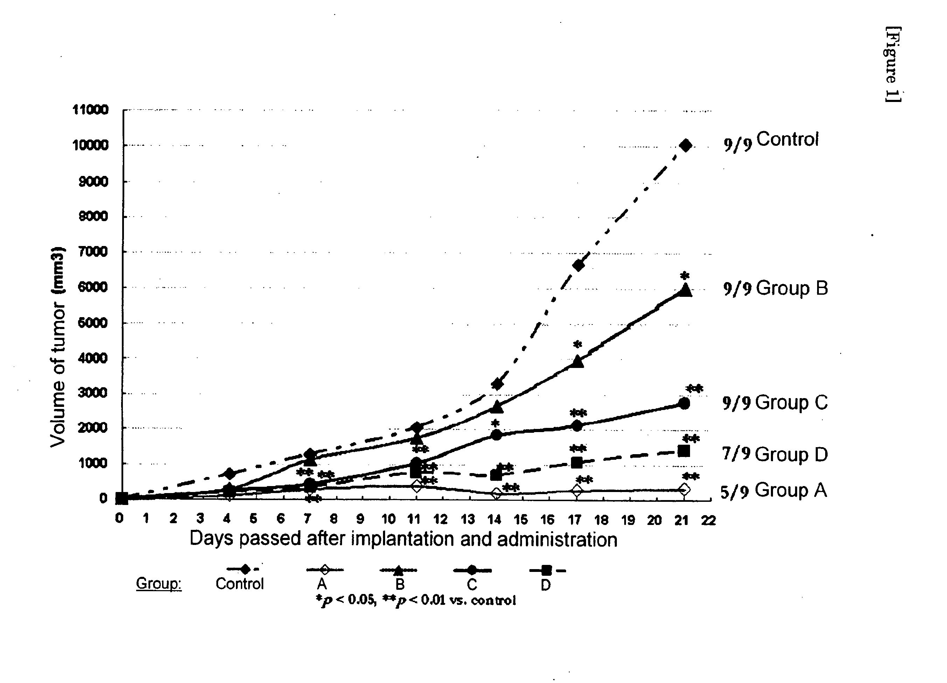

In the test, an anticancer drug 5-FU (20 mg/kg/day) and/or a culture solution of *Aureobasidium sp.* (2 g/kg to 30 g/kg/day) are administered to mice with sarcoma implanted therein. A body weight, the number of leukocytes, and a weight of organs of each mouse are measured as indicators of side effects and obtained values are compared to each other. Immediately after sarcoma is implanted, each test sample is orally administered by having each test animal freely drink the sample as drinking water from a bottle for water absorption. Table 1 shows allocation of the test animals and the average daily dose of the anticancer drug and/or culture solution of the *Aureobasidium sp..* The results of the test are shown in FIG. 1 to FIG. 4.

**[Table 1]**

| Average doses of the anticancer drug and/or the Aureobasidium culture solution | | | |
|---|---|---|---|
| Test group | Tested substance | Number of animals (*n*) | Average dose (mg/kg) |
| Control group | Physiological saline | 9 | - |
| Group A | Anticancer drug (5-FU) | 9 | 19.7±2.7 |
| Group B | Aureobasidium culture solution | 9 | 28,700±1,700 (430.5±22.5)^{*1} |
| Group C | Anticancer drug (5-FU) + Aureobasidium culture solution | 9 | 19.0±1.6 + 1,900±200 (28.5±2.5)^{*1} |
| Group D | Anticancer drug (5-FU) + Aureobasidium culture solution | 9 | 13.5±1.1 + 13,500±1,100 (202.4±16.3)^{*1} |

| | | | |
|---|---|---|---|
| *1: Converted score of solid phase content (1.5%) (mg/kg) | | | |

### Result 1 (Effect of avoiding toxic death by anticancer drugs)

The ordinate axis of the FIG. 1 represents volumes of tumors and the abscissa axis represents the number of days after start of the administration of the anticancer drugs. Allocation of test groups is the same as that showed in Table 1. As seen from the data for the group A, a volume of the tumor was significantly restrained by treatment with the anticancer drugs as compared to the result of the control group not having been subjected to any specific treatment. However, in 21 days after start of the treatment with the ant-cancer drugs, 4 out of 9 mice in the test A group in which treatment with the anticancer drug treatment was carried out were dead. On the other hand, there was no example of death in the control group although growth of a tumor was observed.

The above result obviously indicates the characteristics of the treatment with an anticancer drug, and in this type of treatment, the anticancer drug can suppress growth of a tumor, but normal cells are damaged by toxicity of the anticancer drug. This negative aspect spoils the positive result of the treatment with an anticancer drug. However, when the Aureobasidium culture solution (2 g/kg/day) was used concurrently in the test C group, tumor growth was significantly suppressed (p < 0.01), and no test animal dies like in the control group. The dose of 2 g/kg/day corresponds to 30 mg/kg/day in administration with respect to the solid content (1.5%). It means that the Aureobasidium culture solution shows the effect when administered with almost same level of dosage as the anticancer drug. Further, in the test B group in which treatment was carried out only with the Aureobasidium culture solution (29 g/kg/day), the statistically significant anticancer effect (p < 0.05) was confirmed without the death of any test animal.

### Result 2 (Effect of mitigating reduction of body weight associated with administration of an anticancer drug)

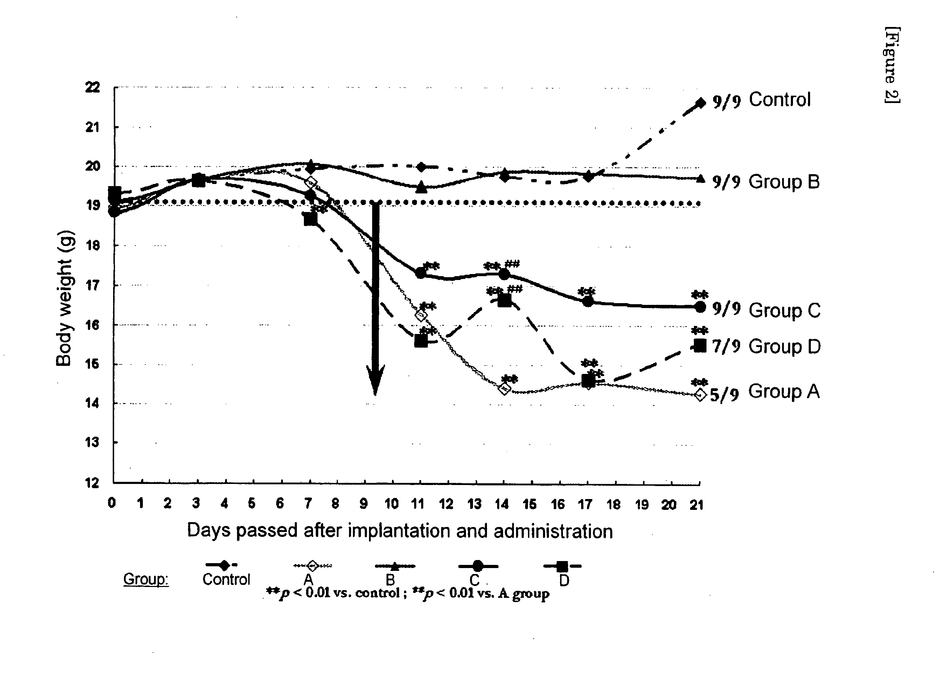

The ordinate axis in FIG. 2 represents the body weight, while the abscissa axis represents the number of days passed after administration of an anticancer drug is started. Allocation of the animal groups is the same as that shown in Table 1. As seen from the data for the test A group, the side effects by treatment with the anticancer drug appeared with reduction of 26% body weight. However, in the test C group in which the Aureobasidium culture solution was used concurrently in treatment with an anticancer drug (by about 2 g/kg/day), the reduction of body weight due to treatment with the anticancer drug could be mitigated to 13%. The mitigation in reduction of body weight caused by side effects of administration of the anticancer drug was recognized as statistically significant different (p<0.05) as compared to a case where treatment was carried out only with the anticancer drug.

### Result 3 (Effect of mitigating reduction of leukocytes associated with administration of an anticancer drug)

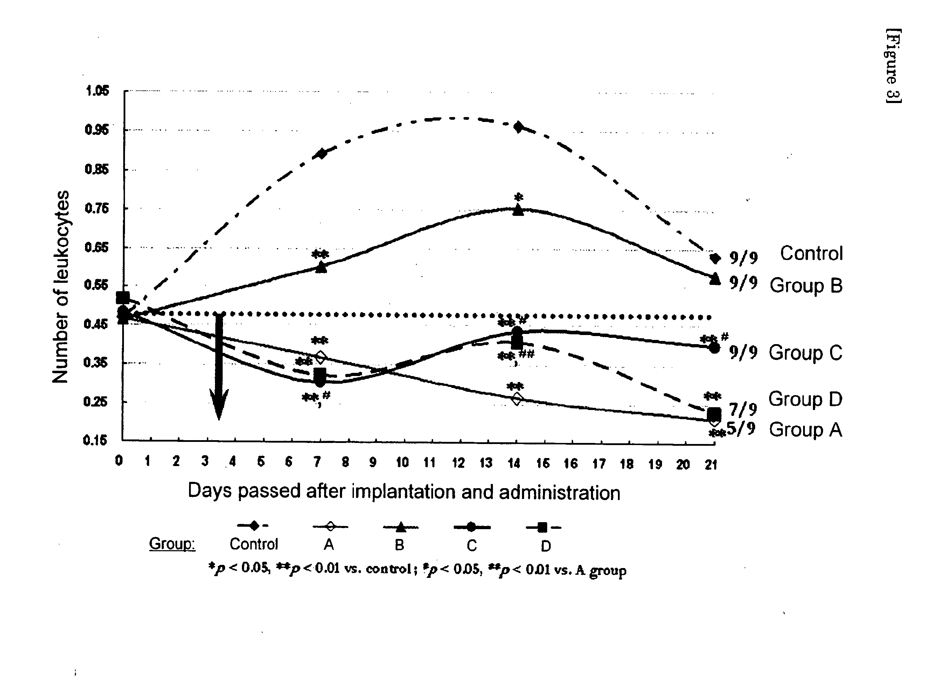

The ordinate axis in FIG. 3 represents the number of leukocytes, while the abscissa axis represents the number of days passed after administration of an anticancer drug is started. Allocation of the animal groups is the same as that shown in Table 1. As seen in the data for the test A group, the side effects by treatment with the anticancer drug appeared with reduction of 42% of the number of leukocytes. However, in the test C group in which the Aureobasidium culture solution was used concurrently in treatment with an anticancer drug (by about 2 g/kg/day), reduction of leukocytes continues for 7 days after start of the treatment, but then the tendency for recovery appeared, and the reduction of leukocytes could be mitigated to 16% in 21 days after start of the treatment. The mitigation in reduction of leukocytes caused by side effects of administration of the anticancer drug was recognized as statistically significant different (p<0.05) as compared to a case where treatment was carried out only with the anticancer drug.

### Result 4: (Effect of mitigating atrophy of organs caused by administration of an anticancer drug)

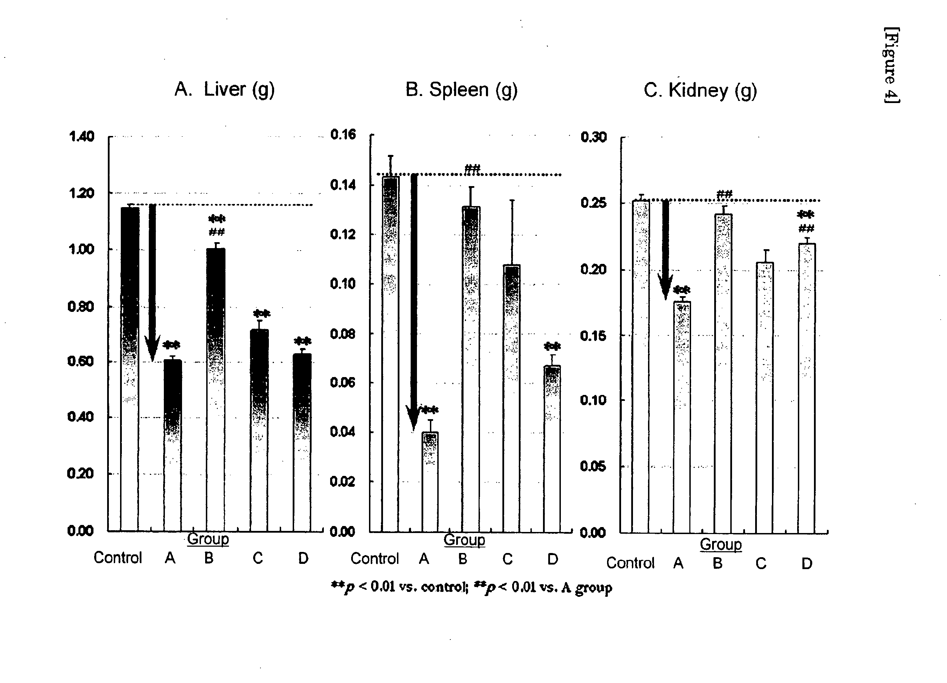

The ordinate axis in FIG. 4 represents weights of organs, while the ordinate axis represents a result of measurement of weights of organs, that is, livers, spleens, and kidneys in each test group in 21 days after start of administration of an anticancer drug. Allocation of the test groups is the same as that shown in Table 1. As seen from the data for the test A group, the side effect of reduction in weights of organs caused in association with treatment with an anticancer drug appeared with reduction of 48% in the livers, of 72% in the spleens, and of 28% in the kidneys. Atrophy of the spleen, which may be regarded as a storage site for lymphocytes, is especially remarkable, and this phenomenon is closely related to the reduction of leukocytes described in Result 3. In the test C group and the test D group, in which the Aureobasidium culture solution was administered concurrently with an anticancer drug, reduction in weights of organs associated with treatment with the anticancer drug could be mitigated.

It is confirmed, based on the result of measurement as described above, that the Aureobasidium culture solution reduces side effects of an anticancer drug and can enhance effects of treatment with the anticancer drug without causing death due to toxicity of the anticancer drug.

### Industrial Applicability

It was confirmed that, by using the Aureobasidium culture solution according to the present invention, damage to tissues caused by the anticancer drug 5-FU can be reduced.

Because the Aureobasidium culture solution according to the present invention can reduce toxicity of an anticancer drug, administration of the drug can be continued in the state where the efficiency is exhibited to the maximum level. Therefore it can be said that the Aureobasidium culture solution is very useful in the clinical field where determination for a life-or-death matter must be made.

## Claims

1. A living body healing accelerator comprising as an active constituent a mixture of a culture solution obtained after cultivation of a microorganism belonging to the *Aureobasidium sp*. and mycelia of the microorganism cultivated in the culture solution, for use *in vivo* in healing damage to tissue caused by the anticancer drug 5-fluorouracil (5-FU) toxicity.

2. A living body healing accelerator according to claim 1, wherein the microorganism belonging to the *Aureobasidium sp.* is the *Aureobasidium pullulans* strain M-1, deposited with number FERM BP-08615 at the International Patent Organism Depositary, National Institute of Advanced Industrial Science and Technology (Independent Administrative Corporation), or the *Aureobasidium pullulans* strain M-2, deposited with number FERM BP-10014 at the International Patent Organism Depositary, National Institute of Advanced Industrial Science and Technology (Independent Administrative Corporation).

3. Use of a mixture of a culture solution obtained after cultivation of a microorganism belonging to the *Aureobasidium sp*. and mycelia of the microorganism cultivated in the culture solution in manufacturing a medicament for healing damage to tissue caused by an anticancer drug, wherein the anticancer drug is 5-fluorouracil (5-FU).

4. A use according to claim 3, wherein the microorganism belonging to the *Aureobasidium sp.* is the *Aureobasidium pullulans* strain M-1, deposited with number FERM BP-08615 at the International Patent Organism Depositary, National Institute of Advanced Industrial Science and Technology (Independent Administrative Corporation), or the *Aureobasidium pullulans* strain M-2, deposited with number FERM BP-10014 at the International Patent Organism Depositary, National Institute of Advanced Industrial Science and Technology (Independent Administrative Corporation).

## Patentansprüche

1. Ein Beschleuniger zum Heilen eines lebendigen Körpers, der als einen wirksamen Bestandteil ein Gemisch einer Kulturlösung umfasst, die nach dem Kultivieren eines Mikroorganismus erhalten wird, der zu den *Aureobasidium sp.* und *mycelia* der Mikroorganismen, die in der Kulturlösung kultiviert werden, gehört, zur *in vivo* Verwendung zum Heilen von Gewebeschäden, die durch die Toxizität des Antikrebsmedikaments 5-Fluoruracil (5-FU) verursacht werden.

2. Ein Beschleuniger zum Heilen eines lebendigen Körpers gemäß Anspruch 1, wobei der Mikroorganismus, der zu dem *Aureobasidium sp.* gehört, der *Aureobasidium pullulans* Stamm M-1 ist, hinterlegt mit der Nummer FERM BP-08615 beim International Patent Organism Depositary, National Institute of Advanced Industrial Science and Technology (Selbstverwaltungskörperschaft) oder der *Aureobasidium pullulans* Stamm M-2 ist, hinterlegt mit der Nummer FERM BP-10014 beim International Patent Organism Depositary, National Institute of Advanced Industrial Science and Technology (Selbstverwaltungskörperschaft).

3. Verwendung eines Gemischs einer Kulturlösung, die nach Kultivieren eines Mikroorganismus, der zu den *Aureobasidium sp.* und *mycelia* der Mikroorganismen, die in der Kulturlösung kultiviert werden, gehört, zur Herstellung eines Medikaments zum Heilen von Gewebeschäden, die durch ein Antikrebsmedikament verursacht werden, wobei das Antikrebsmedikament 5-Fluoruracil (5-FU) ist.

4. Eine Verwendung gemäß Anspruch 3, wobei der Mikroorganismus, der zu dem *Aureobasidium sp.* gehört, der *Aureobasidium pullulans* Stamm M-1 ist, hinterlegt mit der Nummer FERM BP-08615 beim International Patent Organism Depositary, National Institute of Advanced Industrial Science and Technology (Selbstverwaltungskörperschaft) oder der *Aureobasidium pullulans* Stamm M-2 ist, hinterlegt mit der Nummer FERM BP-10014 beim International Patent Organism Depositary, National Institute of Advanced Industrial Science and Technology (Selbstverwaltungskörperschaft).

## Revendications

1. Agent favorisant la guérison d'un organisme vivant comprenant en tant que constituant actif un mélange d'une solution de culture obtenue après culture d'un micro-organisme appartenant à l'espèce *Aureobasidium* et du mycélium du micro-organisme cultivé dans la solution de culture, pour une utilisation *in vivo* pour la guérison d'atteintes au tissu provoquées par la toxicité du médicament anticancéreux 5-fluoro-uracile (5-FU).

2. Agent favorisant la guérison d'un organisme vivant selon la revendication 1, dans lequel le micro-organisme appartenant à l'espèce *Aureobasidium* est la souche M-1 d'*Aureobasidium pullulans,* déposée sous le numéro FERM BP-08615 auprès de l'International Patent Organism Depositary, National Institute of Advanced Industrial Science and Technology (Société Administrative Indépendante), ou la souche M-2 d'*Aureobasidium pullulans,* déposée sous le numéro FERM BP-10014 auprès de l'International Patent Organism Depositary, National Institute of Advanced Industrial Science and Technology (Société Administrative Indépendante).

3. Utilisation d'un mélange d'une solution de culture obtenue après culture d'un micro-organisme appartenant à l'espèce *Aureobasidium* et du mycélium du micro-organisme cultivé dans la solution de culture dans la fabrication d'un médicament pour la guérison d'atteintes au tissu provoquées par un médicament anticancéreux, le médicament anticancéreux étant le 5-fluoro-uracile (5-FU).

4. Utilisation selon la revendication 3, dans laquelle le micro-organisme appartenant à l'espèce *Aureobasidium* est la souche M-1 d'*Aureobasidium pullulans,* déposée sous le numéro FERM BP-08615 auprès de l'International Patent Organism Depositary, National Institute of Advanced Industrial Science and Technology (Société Administrative Indépendante), ou la souche M-2 d'*Aureobasidium pullulans,* déposée sous le numéro FERM BP-10014 auprès de l'International Patent Organism Depositary, National Institute of Advanced Industrial Science and Technology (Société Administrative Indépendante).
